# EUROPÄISCHE PATENTANMELDUNG

(11) **EP 3 300 750 A1**
(43) Veröffentlichungstag der Anmeldung: **04.04.2018**
(21) Anmeldenummer: 16191613.5
(22) Anmeldetag: 29.09.2016
(51) Int. Cl.: A61M 1/10, A61M 1/12

(54) **BLUTPUMPE**

(71) Anmelder: Berlin Heart GmbH, 12247 Berlin (DE)
(72) Erfinder: GRANEGGER, Marcus, 8057 Zürich (CH); WINTERWERBER, Kim Peter, 12357 Berlin (DE)
(74) Vertreter: Pfenning, Meinig & Partner mbB

(57) **Zusammenfassung**

Gegenstand der vorliegenden Anmeldung ist eine Blutpumpe mit einer mechanischen Lageranordnung. Dabei ist ein Kugelsegment vorhanden, welches aus einem Diamant besteht.

## Beschreibung

Gegenstand der vorliegenden Erfindung ist eine Blutpumpe gemäß Anspruch 1 sowie eine Lageranordnung für eine Blutpumpe.

Im Stand der Technik sind verschiedene Möglichkeiten zur Lagerung eines Rotors bei Blutpumpen bekannt. Am gebräuchlichsten sind hierbei mechanische, magnetische, hydrodynamische Lager oder Mischformen der vorgenannten Lager.

Im Falle mechanischer Lager sind sogenannte "Ball/Cup"-Lager bekannt. So beschreibt beispielsweise die US 5 707 218 derartige Lager, bei welchen ein Kugelsegment in einem Kalottensegment sowohl axial als auch radial gelagert ist.

Derartige mechanische Lager haben aufgrund der hohen Anpresskräfte zahlreiche Nachteile.

Aufgabe der vorliegenden Erfindung ist es, eine Lageranordnung für eine Blutpumpe zur Verfügung zu stellen, welche die vorliegenden Nachteile beseitigt.

Erfindungsgemäß wird die Aufgabe gelöst durch den Gegenstand des Anspruchs 1 sowie eine im Anspruch 1 aufgeführte Lageranordnung für eine Blutpumpe.

Die Blutpumpe umfasst ein Gehäuse mit einem stromauf gelegenen Einlass, einem stromab gelegenen Auslass und einem drehbar gelagerten Rotor mit einer Achse und einer Beschaufelung. Ferner ist ein mechanisches Lager zur Lagerung des Rotors vorhanden, wobei das mechanische Lager mindestens ein Kugelsegment und mindestens ein das Kugelsegment zumindest teilweise aufnehmendes Kalottensegment umfasst. Unter einem Kugelsegment wird hierbei ein Kugelabschnitt verstanden, der entweder ein Kugelkörper oder ein Teil eines Kugelkörpers ist, der durch den Schnitt des Kugelkörpers mit einer Ebene gebildet wird. Das Kugelsegment wird auf das Kalottensegment gedrückt, so dass eine formschlüssige Verbindung zwischen den beiden Elementen vorliegt.

Unter einem Kalottensegment wird hierbei die Oberfläche des Inneren eines Kugelabschnitts, d. h. einer Kugelhaube, oder das Innere einer Kugelzone verstanden. Eine Kugelhaube bildet den gekrümmten Teil der Oberfläche eines Kugelsegments. Die Kugelzone bildet den gekrümmten Flächenteil einer Kugelschicht bzw. einer Kugelscheibe.

Bei dem Kalottensegment ist zu beachten, dass die Höhe des Kalottensegments entlang der Rotationsachse nicht größer als ein entsprechender Kugelradius sein darf. Das Kugelsegment und das Kalottensegment sind zumindest abschnittsweise um eine Rotationsachse rotationssymmetrisch, d. h. die aufeinander liegenden bzw. aufeinander gepressten Oberflächen des Kugelsegments und des Kalottensegments sind rotationssymmetrisch. Dabei stimmt die Rotationsachse im Wesentlichen mit einer Achse des Rotors überein.

Das Kugelsegment besteht aus einem vorzugsweise mittels eines durch ein chemisches Abscheideverfahren hergestellten Hartstoff. Unter Hartstoffen werden hierbei beispielsweise Diamanten, Metallcarbide oder bestimmte Keramiken verstanden. Beispielsweise sind neben Diamanten auch Nitride, wie vorzugsweise ein kubisch kristallines Bornitrid, ein Titannitrid oder ein Siliciumnitrid, oder Carbide, wie vorzugsweise ein Siliciumcarbid, ein Borcarbid, ein Wolframcarbid, ein Vanadiumcarbid, ein Titancarbid oder ein Tantalcarbid, und Oxide, wie vorzugsweise ein Aluminiumoxid oder ein Zirconiumdioxid, unter dem Begriff Hartstoffe umfasst. Diese Hartstoffe weisen aufgrund ihrer Härte gute Abriebeigenschaften auf. Ferner besitzen Diamanten eine gute Wärmeleitfähigkeit, so dass die durch Reibung zwischen Kugelsegment und Kalottensegment entstehende Wärme gut durch das die Blutpumpe durchströmende Fluid, z. B. Blut, aufgenommen werden kann. Auch die durch chemische Abscheideverfahren (chemical vapour deposition; CVD) hergestellten Diamanten oder andere der oben aufgeführten Hartstoffe, beispielsweise Siliciumcarbid oder Bornitrid, besitzen den Vorteil, dass sie gegenüber natürlichen Diamanten deutlich kostengünstiger sind und oftmals eine bessere Wärmeleitfähigkeit besitzen. Ferner kann durch moderne formgebende Verfahren die Form des Kugelsegments entsprechend den Lageranforderungen ausgebildet werden.

Das mechanische Lager nimmt vorzugsweise einen Großteil der auf den Rotor wirkenden Axialkräfte auf. Ferner wird vorzugsweise auch ein Teil der radial wirkenden Kräfte durch das Kalottensegment aufgenommen. Die Reibfläche wird dabei durch die Kontaktfläche zwischen dem Kugelsegment und dem Kalottensegment vorgegeben. Hierbei ist jedoch zu beachten, dass zwischen dem Kugelsegment und dem Kalottensegment ein Bereich der elasto-hydrodynamischen Schmierung oder Mischreibung, d. h. eine Reibung durch Festkörper oder Flüssigkeiten, besteht, in welchem eine sehr dünne Schicht Flüssigkeit, z. B., wie im vorliegenden Fall, Blut oder auch eine Kochsalzlösung, vorhanden ist. Die elastohydrodynamische Schmierung unterscheidet sich von einer hydrodynamischen Lagerung.

Um eine geeignete Dimensionierung der Reibflächen zu erreichen, kann vorgesehen sein, dass die Krümmung (d. h. der Radius) des Kugelsegments bzw. des Kalottensegments derart gewählt ist, dass der aus der radial und axial wirkenden Kraft vom Kugelsegment auf das Kalottensegment gegebene Kraftvektor senkrecht auf der Reibfläche steht. Dies kann einen Hinweis auf den zu wählenden Krümmungsradius des Kalotten- bzw. Kugelsegments geben.

Ferner kann die Krümmung des Kugelsegments bzw. des Kalottensegments derart gewählt werden, dass das Verhältnis der Größe der axialen Fläche des Kalottensegments (d. h. die Fläche der Projektion des Kalottensegments in eine Ebene senkrecht zur Rotationsachse) zur radialen Fläche des Kalottensegments (d. h. der Fläche der radialen Projektion des Kalottensegments) derart eingestellt ist, dass dieses dem Verhältnis der axial wirkenden Kraft zur radial wirkenden Kraft entspricht.

Das Verhältnis der Betriebskräfte Axial/Radial (Fa/Fr) definiert vorzugsweise den Winkel einer Starttangente αₛₜₐᵣₜ > arctan(Fa/Fr).

Der Winkel einer Endtangente wird vorzugsweise durch den Reibwert der Materialkombination definiert. Es gilt α_{end} > arctan(µ), unterhalb dieses Winkels gilt die Selbsthemmung, d. h., es besteht die Gefahr überhöhter Reibung.

Vorzugsweise ist die Kalotte so flach (d. h., dass das Verhältnis von Radius quer zur Rotationsachse zur Höhe parallel zur Rotationsachse deutlich größer als 1 ist) auszuführen wie es geht, d. h. der Winkel der Endtangente ist zu maximieren, ohne dass bei auftretendem Verschleiß die Kugel aus der Kalotte hüpfen kann.

Der Kugelradius ist durch den erlaubten Verschleiß und die maximale Oberflächentemperatur nach unten begrenzt. Da die entstehende Wärmemenge theoretisch unabhängig vom Kugelradius ist, wird eine kleine Kugel heißer als eine große.

Die axial und die radial auf das Lager wirkenden Kräfte können beispielsweise durch Strömungssimulationen eines Fluids entlang des Rotors ermittelt werden und die Krümmung bzw. die Fläche des Kugelsegments und/oder des Kalottensegments entsprechend den durch die Simulation ermittelten Kräften gewählt werden.

Im Gegensatz zu Metallen mit einer Diamantbeschichtung, wie Diamond Like Carbon (DLC), besitzt ein mittels eines chemischen Abscheideverfahrens hergestellter Diamant oder anderer Hartstoff deutlich bessere Wärmeleitwerte. Ferner ist ein aus Diamant bestehendes Kugelsegment derart beschaffen, dass das gesamte Volumen des Kugelsegments zum Wärmeaustausch zwischen der Reibungswärme und dem Fluid dienen kann. Weitere Ausführungsformen der Pumpe bzw. der Lageranordnung ergeben sich aus den Unteransprüchen.

In einer Variante der Erfindung ist das Kugelsegment eine Kugel. In einer alternativen Ausführungsform ist das Kugelsegment lediglich ein Kugelabschnitt, wobei der Kugelabschnitt eine Höhe zwischen einem Radius und dem doppelten Radius der Kugel umfassen kann, d. h., das Kugelsegment ist mehr als eine Halbkugel.

Die Kugel bildet das maximale Körpervolumen im Verhältnis zur Körperoberfläche und somit als Reibfläche fungierenden Oberfläche des mechanischen Lagers. Durch das große Volumen der Kugel kann mehr Wärme aufgenommen und Reibungswärme besser an das Blut abgeführt werden.

Im Falle eines Kugelsegments können beispielsweise bei einer Höhe des Kugelsegments entlang der Rotationsachse, welche im Wert zwischen einem halben Radius der Kugel und zwei Radien der Kugel liegt, Kosten aufgrund des geringeren Volumens des Hartstoffs gegenüber dem Volumen einer vollen Kugel gespart werden. Hierbei ergeben sich jedoch Schwierigkeiten, eine exakt ebene Schnittfläche herzustellen. Beispielsweise kann ein als Kugel hergestellter Hartstoff durch ein entsprechendes Verfahren in zwei Halbkugeln geteilt werden (z. B. durch Diamantsägen im Falle einer Diamantkugel). Diese Halbkugeln können dann als Komponenten eines mechanischen Lagers eingesetzt werden.

In einer weiteren Ausführungsform besitzt das Kalottensegment zwischen einem ersten radialen Abstand und einem zweiten radialen Abstand von der Rotationsachse einen zu einem Krümmungsradius des Kugelsegments korrespondierenden Krümmungsradius. Hierdurch greifen das Kalottensegment und das Kugelsegment zumindest abschnittsweise wie ein "Ball/Cup"-Lager ineinander. So kann beispielsweise das Kalottensegment derart beschaffen sein, dass der Krümmungsradius außerhalb des zweiten Abstands, d. h. bei einem größeren Radius als dem zweiten Abstand, größer ist als innerhalb des zweiten Abstands, um beispielsweise auch eine, wenn auch geringe, Spülung des Spalts zwischen dem Kalottensegment und dem Kugelsegment zu ermöglichen.

In einer Ausführungsform ist der Krümmungsradius des Kugel- und des Kalottensegments derart gewählt, dass in einem entlang der Rotationsachse betrachteten Längsschnitt des Kalottensegments der erste Abstand einen ersten Berührungspunkt und der zweite Abstand einen zweiten Berührungspunkt zwischen Kalottensegment und Kugelsegment definiert und eine durch den ersten und zweiten Berührungspunkt definierte Gerade einen Winkel von mehr als 50°, vorzugsweise mehr als 55°, besonders vorzugsweise mehr als 59°, gegenüber der Rotationsachse besitzt. In diesem Ausführungsbeispiel kann beispielsweise der Krümmungsradius zwischen der Rotationsachse und dem ersten radialen Abstand deutlich größer sein, und es kann so eine kleine Mulde bzw. Ausnehmung im Kalottensegment definiert sein. Vom ersten Abschnitt bis zum zweiten Abschnitt ergibt sich dann eine Gerade, welche einen bestimmten Winkel zur Rotationsachse bildet.

Bei zu großen Kontaktwinkeln ist das Lager jedoch nicht mehr in der Lage, die radial wirkenden Kräfte in geeigneter Weise aufzunehmen, und die Effizienz des Lagers verringert sich. Dies betrifft insbesondere Kontaktwinkel von mehr als 80°, so dass der Kontaktwinkel in einigen Ausführungsbeispielen kleiner als dieser Winkel gewählt wird.

In einer weiteren Ausführungsform ist das Kalottensegment in eine Oberfläche eines Zylinders bzw. einer Zylinderhülse eingebettet. Dabei ist das Kalottensegment vorzugsweise in eine der beiden Schnittkreisflächen, d. h. nicht in den Mantel des Zylinders, eingebracht. Die Zylinder- bzw. Zylinderhülsenform hat unter anderem den Vorteil, dass sie auf einfache Art und Weise mit einem Vor- bzw. Nachleitrad oder einem Rotor verbunden werden kann.

In einer weiteren Ausführungsform besteht das Kalottensegment aus dem gleichen Material wie das Kugelsegment. In diesem Fall trägt nicht nur das Kugelsegment, sondern auch das Kalottensegment bzw. die das Kalottensegment beherbergende Zylinderhülse bzw. der Zylinder die aufgrund der Reibung entstehende Wärme auf und gibt diese aufgrund der guten Wärmeleitfähigkeit des Diamanten an das das Lager umströmende Fluid ab. Durch die Länge (d. h. die Länge entlang der Zylinderachse senkrecht zur Schnittfläche) beispielsweise des Zylinders oder der Zylinderhülse kann der Abschnitt, entlang welchem die Wärme bevorzugt abgegeben werden soll, entsprechend eingestellt werden.

In einer weiteren Ausführungsform umfasst die Blutpumpe ein weiteres mechanisches Lager mit einem weiteren Kugelsegment und einem weiteren Kalottensegment. Oftmals ist dieses weitere mechanische Lager an einem entlang der Rotationsachse des Rotors betrachteten gegenüberliegenden Ende des Rotors angeordnet. Das weitere mechanische Lager kann ähnlich wie die hier beschriebenen Lager aufgebaut sein. Dabei kann es jedoch einen größeren Krümmungsradius bzw. einen kleineren oder gleich großen Krümmungsradius wie das erstgenannte mechanische Lager aufweisen.

In einer weiteren Variante ist, für den Fall, dass das weitere mechanische Lager stromabwärts gelagert ist, vorgesehen, dass der Krümmungsradius des Kugelsegments bzw. Kalottensegments größer ist als das stromaufwärts angeordnete Lager. Dies kann sich zum einen positiv auf den Wärmeabtransport durch das Fluid und zum andern auf die Strömungseigenschaften des Fluids auswirken.

In einer weiteren Ausführungsform ist das Kugelsegment mit einem Zapfen, beispielsweise mit einem Metall- oder Keramikzapfen, verbunden, wobei die Verwendung beispielsweise mittels eines adhäsiven Werkstoffs, wie eines Epoxidharzes, vermittelt wird. Der Zapfen ist vorzugsweise in ein Metallelement eingebettet, beispielsweise ein rohrförmiges Metallelement, welches als Teil eines Vor- oder Nachleitrads oder eines Rotors ausgebildet ist. Als Beispiel eines derartigen Epoxidharzes sei das EPO-TEK^{®} 301-2 der Firma Epoxy Technology genannt. Dieses stellt jedoch nur ein Beispiel eines biokompatiblen und nichttoxischen Klebers dar. Im Wesentlichen kann auch auf andere biokompatible Stoffe gemäß dem internationalen Standard ISO 10993 zurückgegriffen werden.

Der Zapfen vereinfacht die Verbindung zwischen dem Kugelsegment und dem eigentlichen Rotor. Ähnlich einem Verbindungsstift bewirkt der Zapfen eine verbesserte und dauerhaftere Verbindung zwischen dem Rotor und dem eigentlichen Kugelsegment. Der Zapfen stellt unter anderem einen Rundlauf des Lagers sicher, wobei der Zapfen derart mit dem Kugelsegment verbunden ist, dass die Rotationsachse des Zapfens durch einen Mittelpunkt des Kugelsegments läuft. Der Mittelpunkt des Kugelsegments entspricht dabei dem Punkt, welcher den Mittelpunkt einer Kugel bildet und an dem die Kugel denselben Radius wie das Kugelsegment besitzt. Der mit dem Kugelsegment verbundene Zapfen wird derart mit dem Rotor verbunden, dass die Rotationsachse des Zapfens koaxial mit der Rotorachse läuft. Dabei ist der Zapfen vorzugsweise drehfest mit dem Kugelsegment verbunden, um keinen ungewollten Lagerabrieb zwischen dem Kugelsegment und dem Zapfen zu erzeugen. Die Verdrehsicherung kann zusätzlich oder alternativ zu einem stoffschlüssigen Verbinden des Kugelsegments mit dem Zapfen verwendet werden und kann beispielsweise durch eine kraft- und/oder formschlüssige Verbindung vermittelt werden.

Um eine verbesserte Wärmeleitfähigkeit zu gewährleisten, kann der Zapfen beispielsweise in ein Metallelement oder ein keramisches Element eingebettet werden. Das einbettende Metallelement bzw. Keramikelement (wie beispielsweise Wolframcarbid, Diamant, Kupfer) ist dabei eine Zylinderhülse bzw. ein rohrförmiges Element, welches den Zapfen umschließt. In einer Ausführungsform kann das einbettende Element beispielsweise aus Titan bestehen.

Ähnlich dem Kugelsegment kann auch das Kalottensegment, insbesondere sofern dieses als Zylinderhülse ausgearbeitet ist, mit einem Zapfen verbunden werden, um das Kalottensegment besser in der Pumpe zu verankern. Auch dieser Zapfen kann von einem metallischen oder keramischen Element eingebettet sein.

In einer weiteren Ausführungsform ist das Kugelsegment drehfest mit einem weiteren, aus einem Diamanten bestehenden Kalottensegment verbunden, Dabei ist das Kugelsegment derart mit dem Kalottensegment verbunden, dass die nicht mit dem drehfest verbundenen Kalottensegment belegte Oberfläche des Kugelsegments in das Kalottensegment des mechanischen Lagers eingreift und mit diesem die Reibfläche bildet. Durch die drehfeste Verbindung mit einem Kalottensegment, welches beispielsweise wie das Kalottensegment des mechanischen Lagers ausgebildet ist, wird eine noch bessere Wärmeabfuhr gewährleistet.

In einer weiteren Ausführungsform ist die Blutpumpe derart ausgestaltet, dass das mechanische Lager derart ausgelegt ist, dass der Reibwert zwischen dem Kugelsegment und dem Kalottensegment geringer als µ = 0,1, vorzugsweise µ < 0,05 ist.

In einer weiteren Ausführungsform ist das Kugelsegment mit einer Vielzahl von Mikrostrukturen belegt. Diese Mikrostrukturen sollen auf der einen Seite dem großflächigen Kontakt zwischen dem Kalottensegment und dem Kugelsegment nicht schaden, jedoch kleine Taschen schaffen, welche die elastohydrodynamische Schmierung verbessern oder vereinfachen. Die Mikrostrukturen können beispielsweise - ähnlich einem Golfball - als Dimples oder Spiralnuten, Loxodrome oder bogenförmige Nuten ausgebildet sein.

Weitere Ausführungsformen können den nachfolgenden Ausführungsbeispielen entnommen werden. Es zeigt
- Fig. 1: eine schematische Darstellung einer Axialpumpe;
- Fig. 2: eine schematische Darstellung einer Axialpumpe mit tangentialem Auslass;
- Fig. 3: eine schematische Darstellung einer weiteren Blutpumpe;
- Fig. 4: eine Variante eines mechanischen Lagers;
- Fig. 5: eine weitere Variante eines mechanischen Lagers;
- Fig. 6: eine Illustration eines Kontaktwinkels;
- Fig. 7: eine weitere Ausführungsform einer Lageranordnung; und
- Fig. 8: eine weitere Ausführungsform einer Lageranordnung

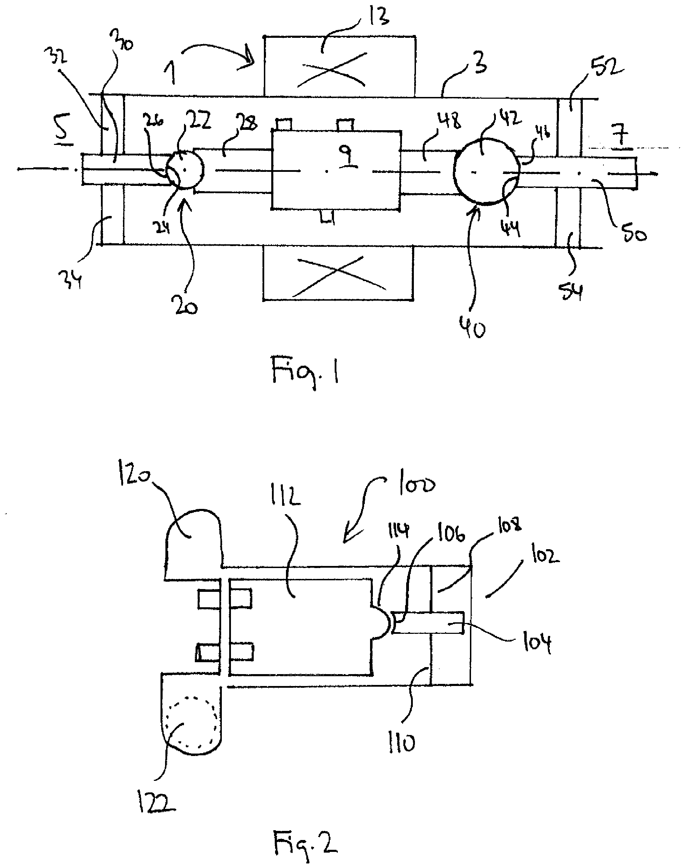

Die Fig. 1 zeigt eine beispielhafte Blutpumpe. Obwohl dies im vorliegenden Beispiel eine Axialpumpe mit einem entlang einer Rotationsachse gelegenen Einlass und Auslass ist, kann es sich bei der Blutpumpe auch um eine Axialpumpe mit radialem Auslass bzw. tangentialem Auslass handeln, wobei der Rotor entweder weiterhin im zylinderförmigen Einlass oder auch im tangentialen Auslass angeordnet ist. Ferner kann die hier vorgestellte Lagerung auch in Radialpumpen zum Einsatz kommen.

Die Blutpumpe 1 umfasst ein Gehäuse 3 mit einem stromaufwärts gelegenen Einlass 5 und einem stromabwärts gelegenen Auslass 7. Zwischen dem Einlass 5 und dem Auslass 7 befindet sich ein Rotor 9, welcher über eine Beschaufelung 11, in diesem Fall eine Wendel, verfügt. Der Rotor beherbergt in seinem Inneren Permanentmagnete, welche mittels eines am Gehäuse 3 außen liegenden Stators 13 in Rotation versetzt werden können. Der Rotor 11 besitzt ferner eine Rotationsachse 15, welche hier im Wesentlichen mit der Achse des zylinderförmigen Gehäuses 3 übereinstimmt.

Die Blutpumpe umfasst ferner ein erstes mechanisches Lager 20, welches eine als Kugel 22 ausgebildete Kugelsegmentfläche 24 besitzt, welche mit einer kalottensegmentartigen Fläche 26 korrespondiert. Die Kugel 22 ist mit dem Rotor über einen Steg drehfest verbunden. Das Kalottensegment 26 ist als Teil eines Vorleitrads 30 ausgebildet und stellt eine Ausbuchtung in dessen Oberfläche dar. Das Vorleitrad 30 wird wiederum über Stege 32 und 34 in dem Gehäuse gehalten. Ferner umfasst die Pumpe ein zweites mechanisches Lager 40, welches ähnlich aufgebaut ist. Das heißt, das Lager umfasst ein als Teil einer Kugel 42 ausgebildetes Kugelsegment 44, welches mit einem Kalottensegment 46 korrespondiert. Die Kugel wird wiederum über einen Steg 48 mit dem Rotor verbunden, während das Kalottensegment 46 als Teil eines Nachleitrades 50 an dessen Oberfläche ausgebildet wurde. Auch das Nachleitrad wird über Stege 52 und 54 im Lumen des Gehäuses gehalten.

In der hier gezeigten Ausführungsform nehmen das vordere und das hintere mechanische Lager, d. h. das erste mechanische Lager und das zweite mechanische Lager, sowohl Axialkräfte, d. h. Kräfte entlang der Rotationsachse 15, als auch Radialkräfte, d. h. Kräfte senkrecht zur Rotationsachse 15, auf.

Wie eingangs erwähnt, kann die Lagerung auch in anderen Pumpen zum Einsatz kommen. Dies sei anhand der alternativen in der Fig. 2 dargestellten Pumpe erläutert. Bei der Blutpumpe 100 handelt es sich um eine Axialpumpe mit einem tangentialen Auslass. Der Einlass 102 beherbergt unter anderem ein Vorleitrad 104, welches ein an einem Fortsatz des Vorleitrades angeordnetes Kalottensegment 106 umfasst. Das Vorleitrad 104 wird über Stege 108 bzw. 110 innerhalb des Gehäuses gehalten. Der Rotor 112 umfasst neben einer Beschaufelung und Permanentmagneten, welche den Rotor mittels eines Stators in Rotation versetzen, an einem stromaufwärtigen Ende des Rotors ein Kugelsegment 114, welches mit dem Kalottensegment 106 korrespondiert. In seinem stromabwärts gelegenen Ende ist der Rotor 112 mittels einer Permanentmagnetlagerung passiv gelagert. Eine den Rotor tangential umlaufende Spiralkammer 120 besitzt einen Auslass 122, welcher stromabwärts des Einlasses 102 liegt. Im vorliegenden Ausführungsbeispiel handelt es sich um eine mit lediglich einem mechanischen Lager ausgebildete Pumpe, welche zudem über eine stromabwärts gelegene radiale passive Magnetlagerung verfügt.

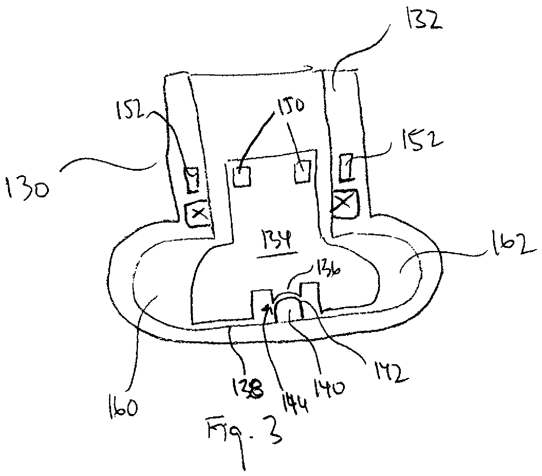

Die Fig. 3 zeigt eine weitere Variante einer Blutpumpe mit einem anmeldungsgemäßen Hartstofflager. Die Pumpe 130 umfasst ein Gehäuse 132, in welchem ein Rotor 134 gelagert ist. Der in der Figur 3 skizzierte Rotor kann entweder eine Radialbeschaufelung und/oder eine Axialbeschaufelung umfassen. Der Rotor 134 weist auf seiner stromabwärts gelegenen Rückseite eine Einbuchtung oder Ausnehmung auf, in welcher das Kalottensegment 136 eines mechanischen Lagers angeordnet ist. An der der Rückseite gegenüberliegenden Wand 138 des Gehäuses 132 befindet sich ein Fortsatz 140, welcher an seinem stromaufwärts gelegenen Ende ein Kugelsegment 142 aufweist, welches mit dem Kalottensegment 136 des Rotors korrespondiert. In einer alternativen Ausführungsform ist das Kugelsegment am Rotor und das Kalottensegment am Gehäuse angeordnet.

Neben dem mechanischen Lager 144, welches das Kalottensegment 136 und das Kugelsegment 142 umfasst, sind am stromaufwärts gelegenen Ende des Rotors 134 Permanentmagnete 150 angeordnet. Die Permanentmagnete 150 bilden zusammen mit einem Ringmagnet oder einer Vielzahl von Permanentmagneten 152 ein passives Magnetlager, welches Radialkräfte und/oder Axialkräfte aufnehmen kann. Bei einem Axiallager sind die Pole der Permanentmagnete parallel zur Rotationsachse ausgerichtet. Bei einem Radiallager können die Pole auch senkrecht zur Rotationsachse ausgerichtet sein.

In dieser Ausführungsform ist in einigen Ausführungsbeispielen kein stromaufwärtiges (d. h. einlassseitiges) Vorleitrad vonnöten. Ferner kann auch auf ein stromabwärtiges Nachleitrad verzichtet werden. In der dargestellten Variante ist kein Vor- oder Nachleitrad vorhanden. Unabhängig vom passiven Magnetlager kann der Rotor weitere Permanentmagnete umfassen, welche zur Ankopplung an den Motorstator 154 dienen.

Der Auslass 160 umfasst eine Spiralkammer oder Volute 162, so dass das Blut in einigen Ausführungsbeispielen tangential zur Einlassrichtung ausfließen kann.

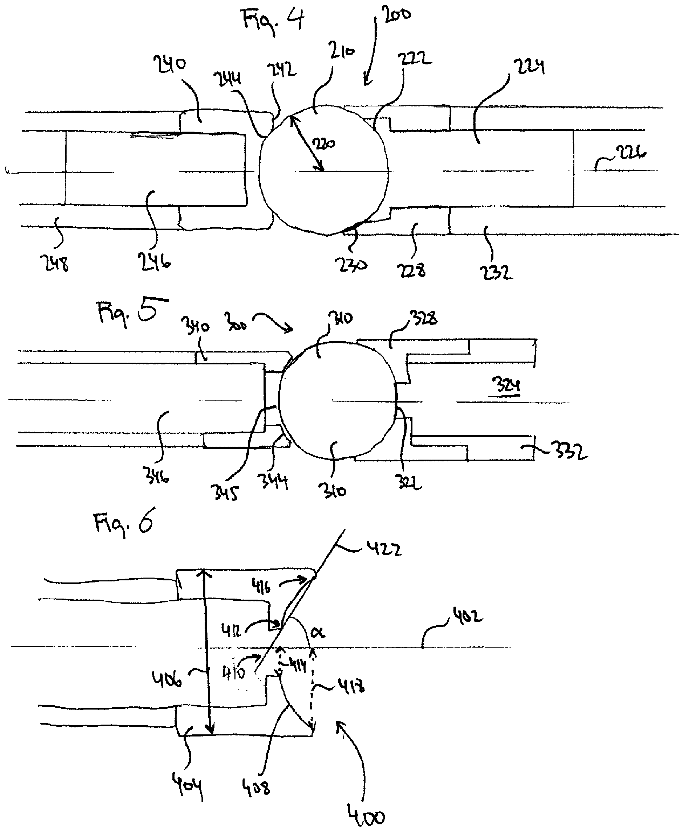

Anhand der Fig. 4 soll eine Ausführungsform eines mechanischen Lagers näher erläutert werden. Das Lager 200 umfasst eine Kugel 210, welche einen Radius 220 von 1 mm besitzt. Die Kugel 210 ist aus einem Diamant, welcher mittels eines CVD-Verfahrens hergestellt wurde. Die Kugel 210 ist mittels eines Epoxidklebers 222 mit einem Keramikzapfen 224 verbunden. Der Keramikzapfen besitzt hierbei eine Länge von 1 cm und wird beispielsweise in einer Achse des Rotors verankert. Die Länge erstreckt sich entlang der Rotationsachse 226. Ferner ist eine Zylinderhülse 228 mit einem kugelzonenförmigen Abschnitt 230 vorhanden, wobei der Kugelzonenabschnitt 230 den gleichen Krümmungsradius wie die Kugel 210 aufweist. Der zylinderhülsenförmige Abschnitt 228 besteht ebenfalls aus einem Diamant. An die Zylinderhülse anschließend befindet sich eine metallische Umhüllung, welche bereits einen Teil des Rotors darstellt.

Die Kugel 210 besitzt einen dem Radius 220 entsprechenden Krümmungsradius. Ferner ist der Abschnitt des Lagers 200, welcher fest mit der Kugel 210 verbunden ist, direkt an den Rotor angekoppelt. Das heißt, dass sich die Kugel 210 bei Rotation des Rotors um die Rotationsachse 226 dreht. Da sowohl die Kugel 210 als auch die Zylinderhülse 228 aus einem Diamant bestehen, besitzt diese Ausführung des mechanischen Lagers gute Wärmeableitungseigenschaften. Da die Zylinderhülse 228 durch den Epoxidkleber 222 ebenfalls fest, d. h. stoffschlüssig, mit der Kugel 210 verbunden ist, ist die Zylinderhülse drehfest mit der Kugel verbunden. Der statische Teil (d. h. der nicht drehende Teil) des mechanischen Lagers 200 umfasst eine weitere zylinderförmige Hülse 240, welche an der Schnittfläche 242 ein Kalottensegment 244 umfasst. Das Kalottensegment ist dabei in Form einer Kugelhaube ausgebildet, deren Krümmungsradius mit dem Krümmungsradius der Kugel 210 übereinstimmt. In eine Öffnung des Zylinders 240 ist ein Zapfen 246 eingeschoben und fest mit diesem verbunden. Der Zapfen kann beispielsweise aus einer Keramik oder aus einem Metall, wie beispielsweise Titan, bestehen. Im Anschluss an den Zylinder 240 befindet sich eine metallische Umhüllung 248, welche beispielsweise Teil eines Vorleitrads sein kann. Ferner kann die metallische Hülse 248 auch Teil eines Nachleitrads sein.

Eine weitere Ausführungsform eines mechanischen Lagers ist in der Fig. 5 gezeigt. Das Lager der Fig. 5 umfasst dabei ebenfalls eine Kugel 310 mit einem Radius 320, welcher im vorliegenden Fall 3 mm beträgt. Die Kugel ist in einem Bereich 322 mit einem keramischen Zapfen 324 verklebt. Der keramische Zapfen weist eine Länge von 0,5 cm auf. Die Kugel 320 wird ferner von einer zylindrischen Hülse 228 eingerahmt, welche im vorliegenden Ausführungsbeispiel aus einer Keramik besteht. Die Kugel 310 besteht aus einem Diamant. Die Hülse 332 ist Teil des Rotors und drehfest mit diesem verbunden. Der statische Teil des mechanischen Lagers umfasst einen Zylinder 340, welcher über ein Kalottensegment 344 verfügt, und weist ferner eine Öffnung 345 auf, so dass sich zwischen dem Zapfen 346 und der Kugel 310 ein Spalt 347 befindet. Dieser Spalt kann hydrodynamische Vorteile haben. Da die Kugel 310 jedoch nur einen sehr geringen Abstand vom Kalottensegment 344 besitzt, beispielsweise einen Abstand von 5 µm aufweist, ist im Bereich der Kontaktfläche zwischen der Kugel 310 und dem Kalottensegment 344 lediglich eine elastohydrodynamische Schmierung vorhanden.

Die Kontaktfläche, welche im Wesentlichen durch das Kalottensegment 344 definiert wird, beträgt im vorliegenden Fall beispielsweise 2 mm². Im Ausführungsbeispiel der Fig. 4 ist die Kontaktfläche deutlich größer und kann beispielsweise 4 mm² betragen. Im Wesentlichen wird die Größe jedoch durch die Größe der Kugel bzw. des Kugelsegments und die Breite der Kugelzone bzw. der Kugelhaube des Kalottensegments bestimmt.

In der Fig. 6 ist ein statischer Teil eines mechanischen Lagers 400 dargestellt, welcher sich entlang einer Rotationsachse 402 erstreckt. Der statische Teil umfasst einen Zylinder 404, welcher einen Durchmesser 406 von 2 mm besitzt. Das Kalottensegment 408 weist einen kreisrunden Ausschnitt 410 auf, welcher einen Durchmesser von 0,5 mm besitzt. In dem hier dargestellten Längsschnitt (auch die übrigen Darstellungen dieser Anmeldung sind Längsschnitte entlang der Rotationsachse 402) beginnt das Kalottensegment am Punkt 412, welcher einen Abstand 414 senkrecht zur Rotationsachse 402 aufweist. Das Kalottensegment endet am Punkt 416, welcher einen Abstand 418 zur Rotationsachse 402 aufweist. Durch die Punkte 412 und 416 wird eine Gerade 422 definiert, welche einen Winkel α mit der Rotationsachse bildet. Der Abstand 414 beträgt, wie eingangs erwähnt, 0,025 mm. Der Abstand 418 beträgt 0,9 mm. Der Radius R des Kalottensegments 408 entspricht der einer Kugel mit einem Radius von 1,1 mm. Aus den Abständen 414, 418 und dem Radius lässt sich so gemäß der Formel O = π(2 R H) die Oberfläche der Kugelzone berechnen, wobei sich die Höhe H aufgrund dessen, dass die Abstände senkrecht auf der Rotationsachse stehen, nach dem pythagoreischen Satz berechnen lassen. Die radiale Projektion ergibt sich aus der Höhe multipliziert mit dem zweifachen Abstand 414 mal π. Die axiale Projektion ergibt sich aus der Kreisfläche, welche durch das Quadrat des größeren Abstands 418 mal π bestimmt wird.

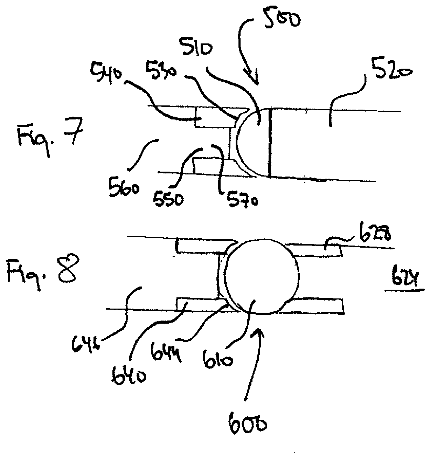

Anhand der Fign. 7 und 8 soll auf weitere Details eines mechanischen Lagers eingegangen werden. So zeigt beispielsweise die Fig. 7 ein mechanisches Lager 500 mit einem Kugelsegment 510, welches in Form einer Halbkugel ausgestaltet ist. Die ebene Fläche der Halbkugel 510 wird mit einem Zapfen 520 drehfest verklebt. Der statische Teil des Lagers umfasst ein Kalottensegment 530, welches als Teil der Schnittfläche einer Zylinderhülse 540 ausgebildet ist. In die Zylinderhülse ist eine Öffnung 550 eingebracht, in welche ein Zapfen 560 mit einem Fortsatz 570 eingreift. Das Kugelsegment, welches als Halbkugel ausgebildet ist, weist einen Radius auf, welcher dem Radius des Kalottensegments 530 entspricht. Die Halbkugel ist aus einem Diamant. Die Zylinderhülse 570 mit dem Kalottensegment 530 kann ebenfalls aus einem Diamant, einer Keramik oder einem Metall hergestellt werden. Dabei ist es besonders vorteilhaft, wenn die Keramik oder das Metall beispielsweise mit einer diamantartigen Karbonschicht (DLC) belegt ist.

Die in der Fig. 8 dargestellte Ausführungsform eines mechanischen Lagers 600 entspricht im Wesentlichen der des in der Fig. 4 dargestellten Lagers. So umfasst der sich drehende Teil eine Kugel 610, welche mit einem Zapfen 624 verbunden ist. Ferner ist die Kugel mit einer Zylinderhülse 628, welche aus Diamant besteht, drehfest verbunden. Korrespondierend zum drehenden Teil des mechanischen Lagers weist der statische Teil des Lagers eine zylinderförmige Hülse 640 mit einem Kalottensegment 644 auf. Gehalten wird die Zylinderhülse 640 von einem Zapfen 646, welcher im vorliegenden Beispiel aus Titan gefertigt ist.

Weitere Ausführungsbeispiele ergeben sich für den Fachmann in naheliegender Weise.

## Patentansprüche

1. Blutpumpe, vorzugsweise zur Unterstützung eines Herzens, wobei die Blutpumpe ein Gehäuse mit einem stromauf gelegenen Einlass, einem stromab gelegenen Auslass und einem drehbar gelagerten Rotor mit einer Achse und einer Beschaufelung umfasst und ein mechanisches Lager zur Lagerung des Rotors vorhanden ist und das mechanische Lager zumindest ein Kugelsegment und zumindest ein das Kugelsegment zumindest teilweise aufnehmendes Kalottensegment umfasst und das Kugelsegment und das Kalottensegment zumindest abschnittsweise um eine Rotationsachse rotationssymmetrisch sind,
wobei
das Kugelsegment aus einem, vorzugsweise mittels eines durch ein chemisches Abscheideverfahren hergestellten Hartstoff besteht.

2. Blutpumpe nach Anspruch 1, wobei der Hartstoff aus der Gruppe der nachstehenden Materialien gewählt ist:
- ein Diamant;
- ein Nitrid, vorzugsweise ein kubisch kristallines Bornitrid, ein Titannitrid oder ein Siliciumnitrid;
- ein Carbid, vorzugsweise ein Siliciumcarbid, ein Borcarbid, ein Wolframcarbid, ein Vanadiumcarbid, ein Titancarbid oder ein Tantalcarbid; und
- ein Oxid, vorzugsweise ein Aluminiumoxid oder ein Zirconiumdioxid.

3. Blutpumpe nach einem der vorhergehenden Ansprüche, wobei das Kugelsegment eine Kugel ist.

4. Blutpumpe nach einem der vorhergehenden Ansprüche, wobei das Kugelsegment eine Kugelschicht ist.

5. Blutpumpe nach einem der vorhergehenden Ansprüche, wobei das Kalottensegment zwischen einem ersten Abstand und einem zweiten Abstand von der Rotationsachse einen zu einem Krümmungsradius des Kugelsegments korrespondierenden Krümmungsradius besitzt.

6. Blutpumpe nach Anspruch 5, wobei in einem entlang der Rotationsachse betrachteten Längsschnitt des Kalottensegments der erste Abstand einen ersten Berührungspunkt und der zweite Abstand einen zweiten Berührungspunkt zwischen Kalottensegment und Kugelsegment definiert und eine durch den ersten und zweiten Berührungspunkt definierte Gerade einen Winkel von mehr als 50°, vorzugsweise mehr als 55°, besonders vorzugsweise mehr als 59°, gegenüber der Rotationsachse besitzt.

7. Blutpumpe nach einem der vorhergehenden Ansprüche, wobei das Kalottensegment aus dem gleichen Material wie das Kugelsegment besteht.

8. Blutpumpe nach einem der vorhergehenden Ansprüche, wobei das Kalottensegment aus einem anderen Material als das Kugelsegment besteht.

9. Blutpumpe nach einem der vorhergehenden Ansprüche, wobei ein weiteres mechanisches Lager mit einem weiteren Kugelsegment und einem weiteren Kalottensegment vorhanden ist, wobei das weitere Kugelsegment und/oder das weitere Kalottensegment aus dem gleichen Material wie das Kugelsegment bestehen.

10. Blutpumpe nach Anspruch 9, wobei das weitere Kugelsegment einen größeren Krümmungsradius als das Kugelsegment besitzt.

11. Blutpumpe nach Anspruch 9, wobei das weitere Kugelsegment einen gleichen Krümmungsradius wie das Kugelsegment besitzt.

12. Blutpumpe nach einem der vorhergehenden Ansprüche, wobei das Kugelsegment mit einem Zapfen verbunden ist und der Zapfen vorzugsweise in ein Metallelement eingebettet ist.

13. Blutpumpe nach einem der vorhergehenden Ansprüche, wobei das Kalottensegment mit einem Zapfen verbunden ist und der Zapfen vorzugsweise in ein Metallelement eingebettet ist.

14. Blutpumpe nach einem der vorhergehenden Ansprüche, wobei das Kugelsegment drehfest mit einem weiteren, aus einem Diamanten bestehenden Kalottensegment verbunden ist.

15. Blutpumpe nach einem der vorhergehenden Ansprüche, wobei das mechanische Lager derart ausgelegt ist, dass der Reibwert zwischen dem Kugelsegment und dem Kalottensegment weniger als 0,2 beträgt.

16. Blutpumpe nach einem der vorhergehenden Ansprüche, wobei das Kalottensegment als Teil einer Oberfläche eines Zylinders oder eines Zylindersegments ausgebildet ist.

17. Blutpumpe nach einem der vorhergehenden Ansprüche, wobei das Kugelsegment eine Vielzahl von Mikrostrukturen aufweist.
